# FASCICULE DE BREVET EUROPEEN

(11) **EP 3 151 003 B1**
(45) Date de publication et mention de la délivrance du brevet: **10.03.2021**
(21) Numéro de dépôt: 16191669.7
(22) Date de dépôt: 30.09.2016
(51) Int. Cl.: G01N 30/08, G01N 30/06, B01J 20/28, B01J 20/32, C07C 7/10, C01B 32/907, B01J 20/282, B01J 20/02, B01D 15/22, C23C 16/40, G01N 1/40, B01L 3/00, B01D 15/20, B01J 20/283, B01J 20/10, B01J 20/281, B01J 20/286

(54) **DISPOSITIF ET PROCEDE D'EXTRACTION DE COMPOSES A CYCLES AROMATIQUES CONTENUS DANS UN ECHANTILLON LIQUIDE**
VORRICHTUNG UND VERFAHREN ZUR EXTRAKTION VON BESTANDTEILEN MIT AROMATISCHEN RINGEN, DIE IN EINER FLÜSSIGKEITSPROBE ENTHALTEN SIND
DEVICE AND METHOD FOR EXTRACTING AROMATIC-RING COMPOUNDS CONTAINED IN A LIQUID SAMPLE

(30) Priorité: 02.10.2015 FR 1559405
(43) Date de publication de la demande: 05.04.2017
(73) Titulaire: COMMISSARIAT À L'ÉNERGIE ATOMIQUE ET AUX ÉNERGIES ALTERNATIVES, 75015 Paris (FR)
(72) Inventeur: FOAN, Louise, 31450 POMPERTUTAT (FR); VIGNOUD, Séverine, 38190 BERNIN (FR); RICOUL, Florence, 38950 QUAIX-EN-CHARTREUSE (FR)
(74) Mandataire: Brevalex

(56) Documents cités:
- WO-A1-2015/097282
- FR-A1- 2 988 620
- FR-A1- 2 997 310
- JP-A- 2013 003 065
- LOUISE FOAN ET AL: "A novel microfluidic device for fast extraction of polycyclic aromatic hydrocarbons (PAHs) from environmental waters - comparison with stir-bar sorptive extraction (SBSE)", INTERNATIONAL JOURNAL OF ENVIRONMENTAL ANALYTICAL CHEMISTRY, vol. 95, no. 13, 4 février 2015 (2015-02-04), pages 1171-1185, XP055245518, GB ISSN: 0306-7319, DOI: 10.1080/03067319.2014.994617

## Description

### DOMAINE TECHNIQUE ET ART ANTÉRIEUR

La présente invention se rapporte à un dispositif et à un procédé d'extraction d'analytes contenus dans un liquide en vue de leur analyse, les analytes présentant un ou des cycles aromatiques, tels que les hydrocarbures aromatiques polycycliques (HAP).

On entend par "analytes", des composés organiques présents dans une matrice liquide.

La qualité de l'eau est influencée par la présence de polluants provenant de rejets industriels et domestiques, des transports et de l'activité agricole. La contamination peut être d'ordre chimique (polluants d'origine minérale ou organique) ou microbiologique (virus, bactéries, parasites...). Parmi les contaminants chimiques, on distingue les macropolluants, tels que les nitrates et les phosphates, présents dans le milieu aquatique en concentrations significatives de l'ordre du mg/l, des micropolluants, terme qui rassemble les éléments traces métalliques (ou métaux lourds) et les composés traces organiques (HAP, pesticides, substances pharmaceutiques...), caractérisés par une toxicité même à des concentrations traces (ng/l au µg/l). A l'heure actuelle, la surveillance des écosystèmes aquatiques se fait principalement grâce à des analyses en laboratoire, qui nécessitent un investissement humain et financier conséquent. Les étapes d'échantillonnage, de transport et de stockage sont particulièrement laborieuses et peuvent induire un biais sur les résultats obtenus du fait de pertes ou de contaminations.

Les HAP sont des polluants organiques persistants émis par de multiples sources (trafic automobile, industries, chauffage domestique) et présentent un véritable enjeu sanitaire. En effet, certains sont réputés cancérigènes ou suspectés de l'être et sont donc surveillés dans les eaux.

Les techniques analytiques de laboratoire adaptées à l'analyse des HAP sont la chromatographie en phase gazeuse et la chromatographie liquide haute performance associées respectivement à la spectrométrie de masse et à une détection par fluorimétrie/UV.

Avant l'introduction dans le dispositif analytique, un processus de traitement des échantillons est nécessaire afin de transférer les analytes d'intérêt de leur milieu d'origine vers une forme appropriée pour l'analyse. Le traitement de l'échantillon permet également de concentrer les composés ciblés ainsi que de les séparer d'éventuelles molécules interférentes présentes dans la matrice.

Pour l'extraction des HAP contenus dans l'eau, les techniques les plus utilisées sont l'extraction liquide-liquide, l'extraction sur phase solide (SPE pour Solid Phase Extraction en terminologie anglo-saxonne), la micro-extraction sur phase solide (SPME pour Solid Phase Microextraction en terminologie anglo-saxonne) et la micro-extraction sur barreau (SBSE pour Stir-Bar Sorptive Extraction en terminologie anglo-saxonne). L'extraction liquide-liquide présente l'attrait d'être simple à mettre en œuvre, surtout si l'on dispose d'un système d'agitation automatisé, et elle ne nécessite qu'une période courte d'optimisation. Cependant, cette technique nécessite un nettoyage rigoureux de la verrerie afin d'éviter la contamination des échantillons. D'autre part, elle consomme des volumes de solvants importants, de l'ordre de 25 ml à 300 ml par échantillon qui sont coûteux à l'achat et lors de leur traitement après utilisation. Enfin, l'extraction liquide-liquide ne peut être réalisée que sur des volumes d'échantillons élevés par exemple entre 250 ml et 1 l.

La SPE, la SPME et la SBSE consistent à plonger dans un liquide ou un gaz une phase solide comprenant une phase stationnaire ayant une affinité avec les analytes d'intérêt, ceux-ci vont s'adsorber sur cette phase. Ensuite la phase solide est retirée du liquide ou du gaz et les analytes sont désorbés en vue de leur analyse. Ces techniques permettent de réduire la consommation de solvants, par exemple de 6 ml à 15 ml par échantillon tout en conservant de bons rendements d'extraction et des limites de détection faibles. La SPE sur disques permet également d'accélérer la procédure de traitement de l'échantillon. Cependant, la SPE ne peut être appliquée à des échantillons de faible volume, contrairement à la SPME et la SBSE. Ces techniques sont également peu consommatrices de solvants. Lorsque le conditionnement et la désorption sont réalisés par thermodésorption, la consommation en solvant est même nulle. En revanche, la SPME montre pour l'analyse des HAP des rendements trop faibles et des limites de quantification trop élevées, tandis que la SBSE montre des résultats très satisfaisants.

La SBSE est basée sur l'extraction par sorption de molécules dissoutes en phase aqueuse par un barreau magnétique recouvert de polydiméthylsiloxane (PDMS).

Les composés ciblés par cette technique sont ceux ayant des coefficients de partage octanol/eau supérieurs à 1000 (log K_{ow} > 3), ce qui est le cas des 16 HAP classés prioritaires par l'Agence de protection de l'environnement américaine (USEPA pour United States Environmental Protection Agency).

Ensuite est réalisée une étape de thermodésorption, par exemple en insérant le barreau SBSE dans un tube en inox qui est soumis à l'étape de désorption. Les analytes sont recondensés sur un piège froid avant d'être envoyés par chauffage rapide ver un dispositif d'analyse, par exemple une colonne de chromatographie en phase gazeuse associée à un détecteur.

La technique d'extraction SBSE mettant en œuvre du PDMS requiert un temps de l'ordre de 1 à 2 heures lorsqu'il s'agit de HAP de masses molaires faibles (i.e. à 2-3 cycles aromatiques), mais pour des HAP de masse molaire plus élevée(i.e. 4-6 cycles aromatiques), elle requiert un temps beaucoup plus long, par exemple supérieur à 12 heures.

Afin de réduire les quantités d'échantillon et de solvants utilisés, ainsi que l'intervention humaine, la miniaturisation et l'automatisation des techniques d'extraction de laboratoire ont été entreprises.

En ce qui concerne les techniques de terrain, il existe à ce jour deux types d'appareillage commercialisés pour analyser les HAP dans l'eau :
- Des sondes de spectroscopie UV par fluorescence (Aqua MS®, CONTROS®, HACH-Lange®) dont la mesure n'est pas spécifique, c'est-à-dire que ces appareils mesurent la concentration de l'ensemble des composés aromatiques qui sont fluorescents à une longueur d'onde donnée (λ_{excitation} = 254 nm et λ_{émission} = 360 nm).
- Un dispositif portable de chromatographie en phase gazeuse couplée à la spectrométrie de masse (GC-MS pour Gas chromatography-mass spectrometry en terminologie anglo-saxonne) (HAPSITE commercialisé par Inficon®) muni d'un système d'échantillonnage par SPME qui ne permet de mesurer que les HAP à 2-3 cycles car l'extraction par SPME ne permet pas de préconcentrer les HAP de masse molaire plus élevée avec des rendements suffisants pour que la technique soit sensible.

Le document L. Foan et al. « A novel microfluidic device for fast extraction of polycyclic aromatic hydrocarbons (PAHs) from environmental waterscomparison with stir-bar sorptive extraction (SBSE)", International Journal of Environmental Analytical Chemistry, vol. 95, n°13, 04 Feb. 2015 décrit un dispositif d'extraction de HAP contenus dans l'eau, comportant une chambre fonctionnalisée avec une couche de PDMS. Les taux d'extraction obtenu par ce dispositif pour la plupart des HAPs sont faibles.

### EXPOSÉ DE L'INVENTION

C'est par conséquent un but de la présente invention d'offrir un dispositif d'extraction qui ait une forte affinité avec des composés organiques tels que les HAP.

Le but énoncé ci-dessus est atteint par un dispositif d'extraction d'analytes d'intérêt en phase liquide comportant au moins un support recouvert d'une couche d'adsorption, ladite couche étant du SiOₓC_{y}H_{z} (oxycarbure de silicium) poreux qui assure une extraction des composés tels que les HAP contenus dans une matrice liquide, selon la revendication 1.

Selon d'autres aspects de l'invention, la revendication 10 définit un procédé de fabrication dudit dispositif et la revendication 13 un procédé d'extraction utilisant ledit dispositif.

Les inventeurs ont découvert que le SiOₓC_{y}H_{z} était particulièrement efficace pour extraire les HAP d'un milieu liquide, et plus généralement pour extraire les composés à cycles aromatiques, notamment les composés à cycles aromatiques à coefficient de partage octanol-eau supérieur à 10³. Celui-ci présente une affinité vis-à-vis de ces composés que rend leur extraction par le SiOₓC_{y}H_{z} particulièrement performante comparativement à d'autres matériaux tels que le PDMS qui présente une affinité limitée avec les composés à cycles aromatiques, et en particulier ceux ayant un coefficient de partage octanol/eau relativement faible, c'est-à-dire situé entre 10³ et 10⁵.

Le dispositif d'extraction comporte une chambre d'extraction dont les surfaces intérieures forment le support, la chambre d'extraction comportant une alimentation de ladite zone en liquide contenant les analytes et une sortie d'évacuation de ladite zone. L'échantillon liquide étant injecté dans une chambre fermée, le dispositif est d'usage pratique comparativement à la manipulation d'un barreau.

De plus l'extraction est plus rapide qu'avec d'autres techniques telles que la technique SBSE et ne requiert qu'un petit volume d'échantillon. Cette rapidité d'extraction rend ce dispositif particulièrement adapté à une utilisation portable.

La zone d'extraction comporte des microstructures, par exemple elle comporte des micropiliers, recouvertes de SiOxCyHz poreux.

Avantageusement un dispositif d'analyse, tel qu'une colonne de chromatographie associée à un détecteur, avantageusement une microcolonne de chromatographie associée à un détecteur, est connectée en sortie du dispositif d'extraction, les composés adsorbés par le SiOₓC_{y}H_{z} étant désorbés avant d'être envoyés vers la colonne de chromatographie. La rapidité d'extraction et l'intégration du dispositif d'extraction et du dispositif d'analyse permettent d'obtenir rapidement les résultats d'analyse et ceci peut être réalisé directement sur la zone de prise de l'échantillon.

La présente invention a alors pour objet un dispositif d'extraction d'analytes à cycles aromatiques, lesdits analytes étant contenus dans une phase liquide, ledit dispositif d'extraction comportant un support et une couche d'adsorption étant du SiOxCyHz poreux, ladite couche d'adsorption recouvrant au moins en partie ledit support.

Très avantageusement, les analytes à cycles aromatiques ont un coefficient de partage octanol-eau supérieur à 10³, et de manière préférée le coefficient de partage octanol-eau est compris entre 10³ et 10⁵.

x peut être compris entre 1 et 2 et de manière préférée entre 1,4 et 1,8 ; y peut être compris entre 0,8 et 3 et de manière préférée entre 1 et 2,5 et z peut être compris entre 2,5 et 4,5 et de manière préférée entre 3 et 4,1.

La couche d'adsorption a par exemple une épaisseur comprise entre 50 nm et 2000 nm, de manière préférée entre 50 nm et 1000nm.

La couche d'adsorption a une porosité comprise entre 3 % et 60%, de préférence entre 10 % et 40 % et peut comporter des pores présentant un rayon compris entre 1 nm et 5 nm.

Le support est formé par au moins les parois d'une chambre d'extraction, ladite chambre d'extraction comportant au moins un orifice d'alimentation en phase liquide et au moins un orifice d'évacuation de la phase liquide, lesdits orifices d'alimentation et d'évacuation étant disposés de sorte que la phase liquide entre en contact avec la couche d'adsorption en s'écoulant de l'orifice d'alimentation vers l'orifice d'évacuation.

La chambre d'extraction comportent des microstructures recouvertes au moins en partie par la couche d'adsorption en SiOxCyHz poreux. Les microstructures peuvent être des micropiliers portant la couche d'absorption.

Le dispositif peut comporter un boîtier comprenant la chambre, et un capot fermant la chambre.

La présente invention a également pour objet un système d'analyse comportant un dispositif d'extraction selon l'invention et un dispositif d'analyse des composés extraits par le dispositif d'extraction.

Le dispositif d'analyse peut être une colonne de chromatographie associée à un détecteur, avantageusement une microcolonne de chromatographie associée à un détecteur.

La présente invention a également pour objet un procédé de fabrication d'un dispositif d'extraction selon l'invention, comportant les étapes:
a) réalisation d'un support,
b) formation d'une couche de SiOxCyHz poreux sur le support,

L'étape b) peut être réalisée par dépôt chimique en phase vapeur, avantageusement par dépôt chimique en phase vapeur assisté par plasma.

Lors de l'étape b), un agent porogène peut être mis en œuvre pendant la formation de la couche de SiOxCyHz, par exemple du norbornadiène, et une étape de recuit pour éliminer l'agent porogène a lieu.

Le support peut être formé par les parois intérieures d'une chambre d'extraction, ladite chambre étant réalisée par photolithographie et gravure, avantageusement gravure DRIE.

La présente invention a également pour objet un procédé d'extraction de composés à cycles aromatiques ayant avantageusement des coefficients de partage octanol/eau supérieurs à 10³ contenus dans une phase liquide, mettant en œuvre un dispositif d'extraction selon l'invention, comportant les étapes:
- mise en contact d'un échantillon liquide comportant au moins un composé aromatique ayant avantageusement un coefficient de partage octanol/eau supérieur à 10³ avec la couche d'absorption

### BRÈVE DESCRIPTION DES DESSINS

La présente invention sera mieux comprise à l'aide de la description qui va suivre et des dessins sur lesquels :
- la figure 1 est une représentation schématique d'un exemple d'un dispositif d'extraction selon l'invention,
- les figures 2A et 2B sont des images de l'intérieur d'un exemple dispositif d'extraction de la figure 1 obtenues par un microscope électronique à balayage à deux grossissements différents,
- la figure 3 est une représentation graphique du rendement d'extraction par un dispositif d'extraction selon l'invention et par la technique SBSE utilisant un barreau magnétique,
- la figure 4 est une représentation graphique d'isothermes d'adsorption de différents HAP représentant l'évolution de la masse de différents HAP adsorbée par masse de phase en fonction de la concentration initiale dans la solution étudiée en µg/L avec un dispositif d'extraction mettant en œuvre une couche de PDMS (courbes en pointillés) et avec un dispositif d'extraction selon l'invention mettant en œuvre une couche de SiOxCyHz (courbes en traits pleins).
- la figure 5 est une représentation graphique de la répartition des pesticides en fonction de la valeur de leur K_{ow},
- la figure 6 est un tableau regroupant plusieurs substances pharmaceutiques avec leur valeur de log K_{ow}.

### EXPOSÉ DÉTAILLÉ DE MODES DE RÉALISATION PARTICULIERS

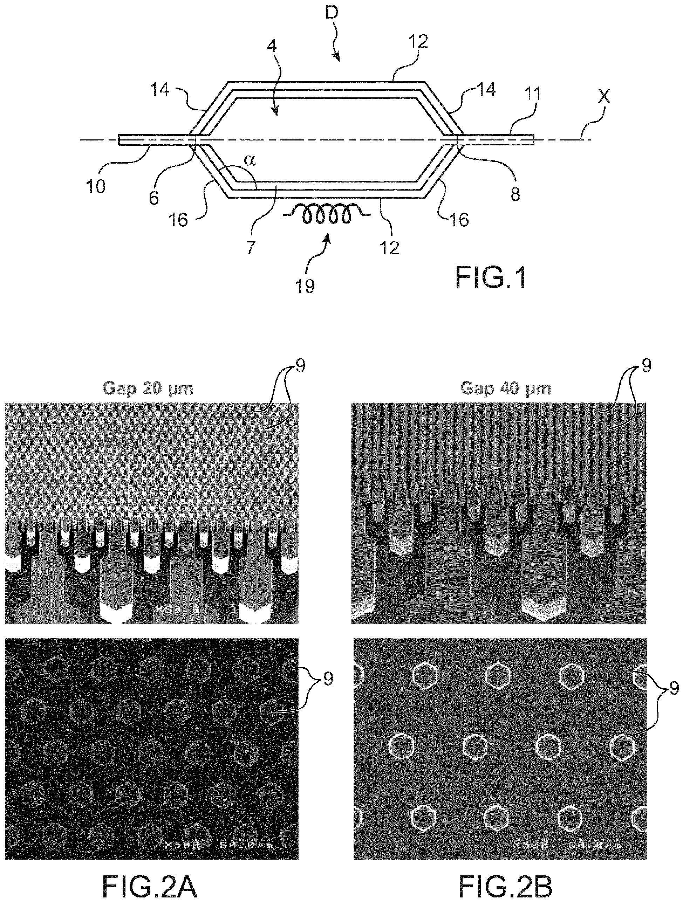

Sur la figure 1, on peut voir une représentation schématique d'un exemple de réalisation d'un dispositif d'extraction selon l'invention.

Le dispositif D comporte une zone d'extraction par absorption et désorption 4 s'étendant le long d'un axe longitudinal X. La zone 4 comporte un orifice d'alimentation 6 et un orifice d'évacuation 8 assurant respectivement l'alimentation de la zone d'extraction 4 en fluide et l'évacuation de la zone d'extraction 4.

A des fins de simplicité, la zone d'extraction par adsorption et désorption sera désignée dans la suite de la description par « zone d'extraction 4 ».

Par exemple, le dispositif D comporte un boîtier et un capot, la zone d'extraction 4 étant réalisée dans le boîtier et est fermée de manière étanche par le capot.

La zone d'extraction 4 comporte une chambre munie d'une surface intérieure sur laquelle les analytes vont s'adsorber et se désorber. La zone d'extraction 4 présente de préférence un grand rapport surface sur volume permettant d'adsorber efficacement les analytes.

La surface intérieure de la chambre est recouverte au moins en partie d'une couche de SiOₓC_{y}H_{z} poreux sur laquelle les composés vont s'adsorber.

Dans cet exemple la zone d'extraction s'étend en trois dimensions, mais il sera compris que selon l'invention la zone d'extraction peut être une zone s'étendant dans deux dimensions et recouverte d'une couche de SiOxCyHz, par exemple une plaque ou lame qui serait destinée à être plongée dans un liquide contenant le ou les analytes à adsorber. Le liquide est une solution aqueuse.

De préférence, x est compris entre 1 et 2, et de manière préférée entre 1,4 et 1,8 ; y est compris entre 0,8 et 3, et de manière préférée entre 1 et 2,5, et z est compris entre 2,5 et 4,5, et de manière préférée entre 3 et 4,1.

La couche de SiOxCyHz a par exemple une porosité entre 3 % et 60%, de préférence entre 10% et 40%. Ce pourcentage représente le volume des pores pour un volume de matériau donné. De préférence les pores présentent un rayon compris entre 1 nm et 3 nm, voire entre 1 nm et 5 nm. Ces dimensions ont été observées par ellipso-porosimétrie, la molécule sonde étant le toluène, le dispositif de mesure étant le modèle EP12 de la société SOPRA.

La couche SiOₓC_{y}H_{z} a par ailleurs de préférence une épaisseur comprise entre 50 nm et 2000 nm, de manière préférée entre 50 nm et 1000 nm.

De manière avantageuse, la zone d'extraction 4 comporte des microstructures permettant d'offrir une grande surface d'adsorption.

Ces microstructures s'étendent de préférence à partir d'un fond inférieur de la chambre. Ces microstructures sont par exemple des micropiliers 9.

Sur les figures 2A et 2B, on peut voir des photos réalisées au microscope électronique à balayage d'une zone d'adsorption comportant des micropiliers. L'espacement entre les piliers est par exemple de 20 µm et 40 µm sur les figures 2A et 2B respectivement, et de préférence compris entre 10 µm et 100 µm, et les micropiliers ont une dimension transversale de préférence comprise entre 10 µm et 100 µm, par exemple égale à 20 µm.

De préférence, les micropiliers ont une section suivant un plan perpendiculaire à l'axe des micropiliers telle qu'elle limite les perturbations dans l'écoulement. Dans l'exemple représenté sur les figures 2A et 2B, les micropiliers ont une section hexagonale. De préférence, les piliers sont orientés de sorte qu'ils soient dans la direction du flux symbolisée par la flèche F afin de limiter les pertes de charge. Dans l'exemple représenté, les plots présentent deux faces sensiblement parallèles au flux et quatre faces inclinées le long desquelles le flux s'écoule. En variante, les micropiliers pourraient avoir une section carrée ou toute autre section offrant une surface importante. Cette forme est en outre compatible avec les procédés de fabrication par les techniques de la microélectronique.

Néanmoins il sera compris que les micropiliers peuvent présenter une section ayant une autre forme, par exemple carrée ou circulaire, sans sortir du cadre de la présente invention.

La couche d'adsorption 7 est portée par les microstructures et par la surface des parois de la chambre. Ces microstructures augmentent la surface sur laquelle les analytes peuvent s'adsorber, ce qui permet d'augmenter la sensibilité de l'analyse.

La zone d'extraction présente alors une surface de capture suffisante tout en limitant les pertes de charge du flux de liquide.

La configuration de la chambre et en particulier de la face dans laquelle débouche l'orifice d'alimentation sont telles qu'elles assurent une bonne distribution du liquide dans la chambre afin d'avoir un bon contact entre le liquide et la couche d'adsorption 7.

L'alimentation de la chambre se fait au moyen d'un capillaire 10 connecté à l'orifice d'alimentation 6 et l'évacuation de la chambre se fait au moyen d'un capillaire 11 connecté à l'orifice d'évacuation 8. Dans l'exemple représenté et de manière avantageuse, la chambre a la forme d'un hexagone présentant deux côtés de plus grande dimension 12, parallèles à l'axe longitudinal.

De préférence, l'orifice d'alimentation 6 et l'orifice d'évacuation 8 sont situés à deux sommets opposés de l'hexagone reliant deux côtés 14, 16 respectivement. Les orifices 6 et 8 se trouvent alors avantageusement sur l'axe longitudinal X, ce qui permet d'obtenir de bonnes performances hydrodynamiques. Ainsi, le trajet d'écoulement est le plus long possible dans la chambre et la distribution du fluide entre les microstructures est favorisée par la forme évasée délimitée par les côtés 14.

La distance entre les micropiliers et la longueur de la chambre, ainsi que leurs formes sont choisies pour permettre à la fois une bonne extraction des analytes d'intérêt contenus dans l'échantillon et le séchage de la chambre. De préférence, la longueur de la chambre est plus grande que sa largeur pour favoriser la distribution du liquide entre les micropiliers et augmenter le temps de contact entre le liquide et la phase adsorbante. Le rapport entre la longueur et la largeur de la chambre est par exemple compris entre 1 et 3.

Par exemple, la zone d'extraction 4 peut comporter avantageusement des moyens de chauffage 18, représentés schématiquement, de sorte à permettre la désorption des analytes préalablement adsorbées. Par exemple, les moyens de chauffage 18 sont formés par une ou plusieurs résistances électriques, par exemple situées sur une face extérieure du module. Avantageusement des moyens de mesure (non représentés) de la température au sein de la zone d'adsorption sont prévus, par exemple formés par deux résistances électriques.

Le fonctionnement du dispositif va maintenant être décrit.

La zone d'extraction 4 est alimentée avec l'échantillon liquide comportant les analytes d'intérêt via le capillaire 10 et l'orifice d'alimentation 6, l'échantillon circule dans la chambre entre les micropiliers. Il entre en contact avec les micropiliers et la surface intérieure de la chambre, plus particulièrement avec la couche d'adsorption 7. Les analytes sont adsorbés par la couche d'adsorption 7. L'échantillon est ensuite évacué par l'orifice d'évacuation. Le débit dans la chambre est ajusté afin d'optimiser la quantité d'analytes adsorbés. On peut également envisager de faire à nouveau circuler l'échantillon dans la chambre pour augmenter la quantité d'analytes adsorbés.

On peut envisager d'arrêter l'écoulement de l'échantillon dans la chambre pour favoriser le contact, de le ralentir progressivement jusqu'à son arrêt ou de maintenir l'écoulement à travers la chambre.

L'intérieur de la chambre est ensuite avantageusement séché, par exemple en faisant circuler un courant d'hélium, d'air sec ou d'azote.

La chambre comporte donc des analytes adsorbés sur la couche d'adsorption 7.

Lors d'une étape suivante, les analytes sont désorbés en vue de leur analyse, par exemple par une colonne de chromatographie associée à un détecteur. Pour cela, on fait circuler dans la chambre un gaz porteur par exemple l'hélium, l'azote ou l'air sec. Les moyens de chauffage sont ensuite activés, ce qui provoque la désorption des analytes, qui sont alors emportés par le flux de gaz porteur vers la colonne de chromatographie ou tout autre dispositif d'analyse. Dans le cas où une colonne de chromatographie est mise en œuvre, les analytes sont séparés en fonction de leur affinité avec la couche stationnaire recouvrant l'intérieur de la colonne de chromatographie et sont détectés par exemple par un détecteur situé en sortie de la colonne.

L'orifice d'alimentation 6 de la zone d'extraction 4 est avantageusement connecté à une source de gaz porteur et à une source d'échantillons contenant les analytes d'intérêt par une vanne à plusieurs voies. L'orifice d'évacuation de la zone d'extraction est avantageusement connecté au détecteur et à une zone de déchet ou de collecte par une vanne à plusieurs voies. La commutation des vannes peut être à commande manuelle ou à commande automatisée.

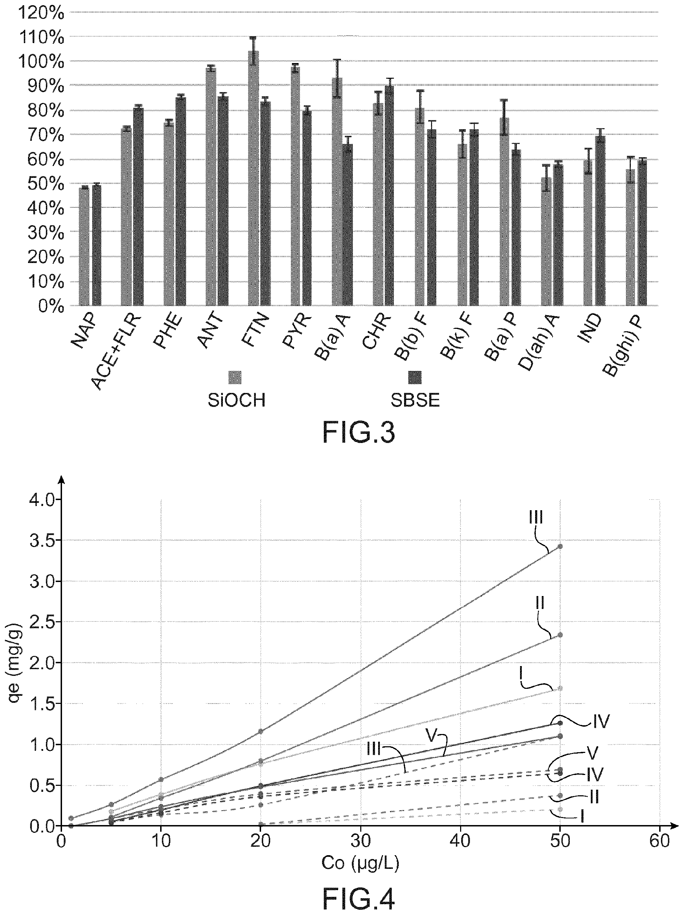

Sur la figure 3, on peut voir un graphique représentant le rendement d'extraction par un dispositif d'extraction selon l'invention et par la technique SBSE utilisant un barreau magnétique recouvert de PDMS.

Le dispositif d'extraction comporte une chambre de surface spécifique de 14 cm². Un échantillon de 10 ml d'eau dopée à 5 µg/l de HAP est analysé. Les analytes ont été récupérés grâce à une désorption par solvant.

On constate que l'on obtient des rendements d'extraction comparables à ceux obtenus avec la technique SBSE, avec un barreau de 1 cm de longueur recouvert d'une couche de PDMS de 0,5 mm d'épaisseur, pour les HAP suivants:
Naphtalène (NAP),
Acénaphtanlène (ACE) + Fluorène (FLR),
Phenantrène (PHE),
Anthracène (ANT),
Fluoranthène (FTN),
Pyrène (PYR),
Benzo(a)pyrène (B(a)A)
Chrysène (CHR),
Benzo(b)fluoranthène (B(b)F),
Benzo(k)fluoranthène (B(k)F),
Benzo(a)pyrène (B(a)P)
Dibenzo(a,h)anthracene (D(ah)A),
Indéno(1,2,3-c,d)pyrène (IND),
Benzo(ghi)perylène (B(ghi)P).

L'extraction par le dispositif selon l'invention est en revanche beaucoup plus rapide que par la technique SBSE utilisant un barreau recouvert d'une couche de PDMS, puisqu'il a fallu environ 20 min avec le dispositif selon l'invention alors qu'il a fallu environ 24 heures avec la technique SBSE. La présente invention permet donc une extraction environ 50 fois plus rapide qu'avec la technique SBSE mettant en œuvre du PDMS.

En outre, le dispositif d'extraction de la figure 1 est beaucoup plus compact et d'utilisation beaucoup plus pratique que la technique SBSE.

Il est donc particulièrement adapté à une utilisation sur le terrain. En outre son intégration avec un dispositif d'analyse est plus aisée.

Sur la figure 4, on peut voir un graphique représentant les isothermes d'adsorption de différents HAP, avec qe la masse de HAP adsorbée par masse de phase en mg/g et **Co** la concentration initiale dans la solution étudiée en µg/l, avec un dispositif d'extraction mettant en œuvre une couche de PDMS (courbes en pointillés) et avec un dispositif d'extraction selon l'invention mettant en œuvre une couche de SiOxCyHz (courbes en traits pleins).

Les courbes I correspondent à l'acénaphtène, les courbes II correspondent à l'anthracène, les courbes III correspondent au fluoranthène; les courbes IV correspondent au benzo(a)pyrène et les courbes V correspondent au indéno(1,2,3-c,d)pyrène).

Afin de montrer la meilleure affinité des HAP envers le SiOxCyHz par rapport au PDMS, des lames de silice de 2.5x2.5 cm² fonctionnalisées avec 150 nm de phase ont été immergées dans 10 ml de solution de HAP sous agitation de 110 tours/minute pendant 24 h. Cinq concentrations ont été testées: 1 µg/l, 5 µg/l, 10 µg/l, 20 µg/l et 50 µg/l. La désorption a été réalisée avec 3 ml d'acétonitrile durant 20 min aux ultrasons. Le solvant a ensuite été analysé par chromatographie liquide haute performance (HPLC), associée à une détection par fluorimétrie (HPLC-FLD).

On constate que la mise en œuvre d'une couche d'extraction en SiOxCyHz selon l'invention permet, par rapport à un dispositif d'extraction utilisant le PDMS, d'obtenir des rendements d'extraction à l'équilibre 2 à 5 fois plus élevés. On constate également que le facteur entre les deux rendements est d'autant plus élevé que les composés sont de masses molaires faibles.

Les HAP suivants sont classés par masse, ce classement correspond à celui des rapports des rendements avec le SiOxCyHz sur ceux avec le PDMS : acénaphtène > anthracène > fluoranthène > benzo(a)pyrène > indéno(1,2,3-c,d)pyrène).

La mise en œuvre d'une couche d'extraction en SiOxCyHz poreux selon l'invention permet donc d'obtenir des rendements d'extraction des composés à 2-3 cycles aromatiques, comme par exemple l'acénaphtène, nettement augmentés par rapport à ceux obtenus par un dispositif d'extraction utilisant le PDMS.

A titre de comparaison des couches d'extraction en silice poreuse ont été testées, celles-ci montrent une affinité pour les HAP bien moins importantes que la couche d'extraction en SiOxCyHz, c'est par exemple le cas d'une couche d'extraction en silice mésoporeuse formée par procédé sol-gel ayant une porosité de 50%. Cette comparaison montre également que l'affinité du SiOxCyHz aux HAP, n'est pas liée uniquement à sa porosité mais à une affinité chimique particulière.

La présente invention est particulièrement adaptée à l'extraction des analytes à cycles aromatiques présentant un coefficient octanol/eau (K_{ow}) supérieur à 10³. Il s'agit notamment des polluants organiques persistants (POP) tels que les HAP, l'HCB (hexachlorobenzene), les PCB (polychlorobiphényles), les PCDD/F (Dioxines et Furanes), les PBDE (Polybromodiphényléthers) et les pesticides organochlorés.

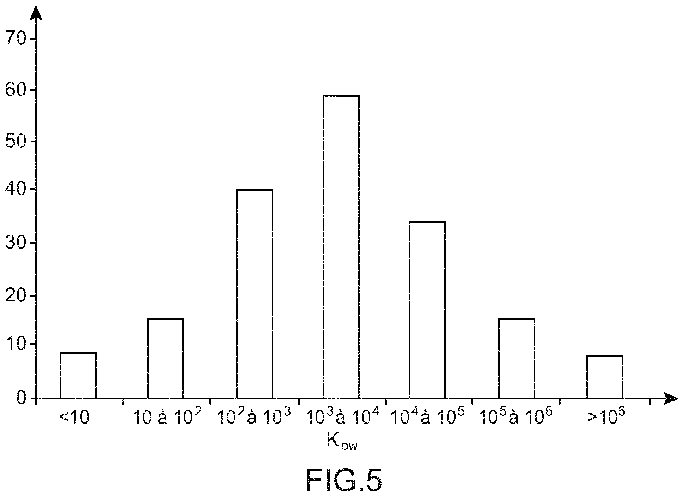

Sur la figure 5, on peut voir la répartition des pesticides en fonction de la valeur de K_{ow}. On constate que la majorité des pesticides a un coefficient K_{ow} compris entre 10² et 10⁵ Par conséquent le dispositif d'extraction selon l'invention est particulièrement efficace pour la détection d'un grand nombre de familles de pesticides. Ce dispositif est donc envisageable pour l'extraction des pesticides de polarité moyenne (K_{ow} de 10³ à 10⁵) ayant peu d'affinité pour le PDMS.

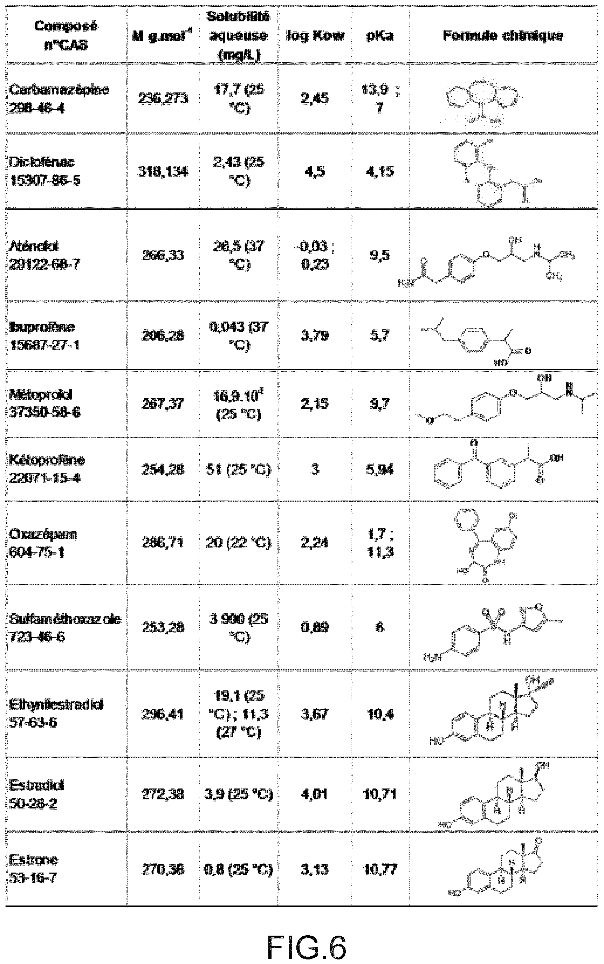

La présente invention s'applique également avantageusement à l'extraction de substances pharmaceutiques comme par exemple le diclofénac, l'ibuprofène, le kétoprofène, l'éthynilestradiol, l'estradiol et l'estrone, qui sont des molécules aromatiques dont le log K_{ow} est compris entre 3 et 4,5. Sur la figure 6, on peut voir différentes substances pharmaceutiques avec leur valeur de log K_{ow} dont celles énumérées ci-dessus et qui peuvent être extraites par le dispositif selon l'invention.

Un exemple de procédé de réalisation d'un dispositif d'extraction selon la figure 1 va maintenant être décrit.

Le dispositif comporte par exemple un boîtier muni de micropiliers en silicium et d'un capot en verre. Le boîtier peut être fabriqué par des techniques classiques utilisées dans le domaine des micro-technologies.

On peut appliquer à un substrat une étape de photolithographie puis une étape de gravure, avantageusement de gravure ionique réactive profonde (DRIE pour Deep Reactive Ion Etching en terminologie anglo-saxonne).

La couche d'adsorption en SiOxCyHz poreux est ensuite formée sur la surface gravée du substrat.

La couche de SiOxCyHz peut avantageusement être déposée par dépôt chimique en phase vapeur ou CVD (Chemical Vapor Deposition en terminologie anglo-saxonne). Le dépôt de SiOxCyHz est alors conforme, i.e. il présente une épaisseur homogène sur toute la longueur des parois du dispositif. L'adjonction d'agents porogènes lors du dépôt permet de maitriser la porosité.

De manière encore plus avantageuse, elle peut être déposée par dépôt chimique en phase vapeur assisté, par exemple par plasma, méthode connue sous l'acronyme ou PECVD (Plasma-Enhanced Chemical Vapor Deposition en terminologie anglo-saxonne), ce qui permet d'effectuer le dépôt à basse température et ainsi de conserver le caractère organique du matériau. L'homogénéité de la distribution spatiale des agents porogènes est favorisée et, in fine, des pores formés lors de l'élimination des agents porogènes.

Par exemple, la couche d'adsorption en SiOxCyHz poreux est réalisée par dépôt chimique en phase vapeur assisté par plasma à partir d'un précurseur organosilicié et d'O₂. Par exemple, on peut utiliser comme précurseurs le TriMethylSilane (3MS), le tetraMethylSilane (4MS), l'OctaMethylCycloTetraSiloxane (OMCTS) et le DimethylDiMEthOxy Silane (DMDMOS). Ces matériaux ont une structure composée de liaisons -Si-O-Si dans laquelle une partie des atomes d'oxygène est remplacée par des groupements méthyles.

Dans un exemple particulier, deux précurseurs ont été utilisés : une matrice organo-silicique de diéthoxymethylsilane et un porogène organique, le norbornadiène. Ainsi lors du dépôt par PECVD, un couche formée d'une matrice contenant des inclusions organique de norbornadiène est obtenue. Les inclusions organiques sont ensuite été éliminées par traitement, par exemple par traitement UV et recuit à 400°C. La couche présente alors une certaine porosité.

Selon une autre variante avantageuse, le dépôt peut être réalisé par dépôt chimique en phase vapeur assisté par filament ou FACVD (Filament Assisted Enhanced Chemical Vapor Deposition en terminologie anglo-saxonne), ce qui permet d'optimiser la conformité.

Selon une autre variante, le dépôt peut être réalisé par dépôt chimique en phase vapeur d'une première couche comprenant le SiOxCyHz avec ou non un agent porogène, suivi par dépôt chimique en phase vapeur d'une deuxième couche de manière à former une deuxième couche étanche au gaz. On réalise ensuite une étape de moussage, qui permet la formation de pores dans la couche de SiOxCyHz. La deuxième couche est ensuite éliminée. Ce procédé est par exemple décrit dans la demande de brevet FR2918997. Il permet l'obtention de pores de taille plus élevée que les dépôts par CVD ou CVD assistés.

En variante encore, la couche de SiOxCyHz peut également être déposée par procédé sol-gel, l'agent porogène étant initialement mélangé dans le sol puis éliminé après gélification.

Par ailleurs, on peut prévoir de faire subir à la couche de SiOxCyHz poreux un post traitement pour modifier les fonctions chimiques de surface. Un tel post traitement permet d'ajuster la sélectivité du matériau par rapport à un analyte donné. Un exemple de post traitement est l'application d'un plasma O₂, N₂ ou N₂O. Un autre exemple est une silanisation, c'est-à-dire le greffage covalent de molécules organiques via une fonction silane.

En fonction de la durée de dépôt, des épaisseurs de SiOxCyHz variables sont obtenues.

Par exemple, on peut choisir une épaisseur de 150 nm, une porosité de 30% mesurée par ellipso-porosimétrie, les pores ayant un rayon moyen de 1,3 nm.

La fonctionnalisation par le SiOxCyHz présente en outre l'avantage d'offrir une meilleure reproductibilité du dispositif d'extraction que la fonctionnalisation par PDMS puisque le dépôt de SiOxCyHz par PECVD permet de réaliser un dépôt plus conforme et d'obtenir une porosité maîtrisée.

Lors d'une étape suivante, le capot est scellé sur le boîtier, par exemple par scellement anodique.

Les connexions microfluidiques sont ensuite réalisées par exemple par des capillaires. Les entrées/sorties sont dans l'axe de la puce.

Une résistance de chauffage, par exemple en Pt est réalisée en face arrière afin de pouvoir procéder à la désorption thermique des analytes adsorbés. Pour cela, on forme préalablement une couche isolante électrique, par exemple en oxyde par exemple du SiO₂, sur la face arrière du substrat puis on forme sur la couche d'oxyde une couche conductrice électrique, par exemple une couche de platine. La couche de platine est ensuite structurée, par exemple par photolithographie et gravure. Avantageusement, on réalise des moyens pour mesurer la température de la puce, par exemple en réalisant deux résistances intégrées en face arrière.

La présente invention porte également sur tout dispositif d'extraction d'analytes d'intérêt à cycles aromatiques selon la revendication 1, de préférence d'analytes à cycles aromatiques dont le coefficient de partage octanol-eau est supérieur à 10³, contenus dans une phase liquide mettant en œuvre une couche d'adsorption en SiOxCyHz poreux, tels que, de manière non limitative, les dispositifs mettant en œuvre les techniques SBSE, SPE et SPME, offrant des rendements d'extraction augmentés et un temps d'analyse réduit.

## Revendications

1. Dispositif d'extraction d'analytes à cycles aromatiques, lesdits analytes étant contenus dans une phase liquide, ledit dispositif d'extraction comportant un support et une couche d'adsorption (7), ladite couche d'adsorption étant de l'oxycarbure de silicium SiOxCyHz poreux, ladite couche d'adsorption recouvrant au moins en partie ledit support, le dispositif d'extraction étant configuré pour que la phase liquide entre en contact avec la couche d'adsorption, dans lequel le support est formé par au moins les parois d'une chambre d'extraction, ladite chambre d'extraction comportant au moins un orifice d'alimentation (6) en phase liquide et au moins un orifice d'évacuation (8) de la phase liquide, lesdits orifices d'alimentation et d'évacuation étant disposés de sorte que la phase liquide entre en contact avec la couche d'adsorption en s'écoulant de l'orifice d'alimentation vers l'orifice d'évacuation, ladite chambre d'extraction comportant des microstructures (9) recouvertes au moins en partie par la couche d'adsorption en oxycarbure de silicium poreux.

2. Dispositif d'extraction selon la revendication 1, dans lequel les analytes à cycles aromatiques ont un coefficient de partage octanol-eau supérieur à 10³, et avantageusement compris entre 10³ et 10⁵.

3. Dispositif d'extraction selon la revendication 1 ou 2, dans lequel x est compris entre 1 et 2 et de manière préférée entre 1,4 et 1,8 ; y est compris entre 0,8 et 3 et de manière préférée entre 1 et 2,5 et z est compris entre 2,5 et 4,5 et de manière préférée entre 3 et 4,1.

4. Dispositif d'extraction selon la revendication 1, 2 ou 3, dans lequel la couche d'adsorption (7) a une épaisseur comprise entre 50 nm et 2000 nm, de manière préférée entre 50 nm et 1000nm.

5. Dispositif d'extraction selon l'une des revendications 1 à 4, dans lequel la couche d'adsorption (7) a une porosité comprise entre 3 % et 60%, de préférence entre 10 % et 40 % et comporte des pores présentant un rayon compris entre 1 nm et 5 nm.

6. Dispositif d'extraction selon l'une des revendications 1 à 5, dans lequel les microstructures (9) sont des micropiliers portant la couche d'absorption.

7. Dispositif d'extraction selon l'une des revendications 1 à 6, comportant un boîtier comportant la chambre, et un capot fermant la chambre.

8. Système d'analyse comportant un dispositif d'extraction selon l'une des revendications 1 à 7 et un dispositif d'analyse des composés extraits par le dispositif d'extraction.

9. Système d'analyse selon la revendication 8, dans lequel le dispositif d'analyse est une colonne de chromatographie associée à un détecteur, avantageusement une microcolonne de chromatographie associées à un détecteur.

10. Procédé de fabrication d'un dispositif d'extraction selon l'une des revendications 1 à 7, comportant les étapes:
a) réalisation d'un support,
b) formation d'une couche d'oxycarbure de silicium SiOxCyHz poreux sur le support.

11. Procédé de fabrication selon la revendication 10, dans lequel l'étape b) est réalisée par dépôt chimique en phase vapeur, avantageusement par dépôt chimique en phase vapeur assisté par plasma et/ou lors de l'étape b), un agent porogène est mis en œuvre pendant la formation de la couche d'oxycarbure de silicium poreux, par exemple du norbornadiène, et une étape de recuit pour éliminer l'agent porogène a lieu.

12. Procédé de fabrication selon la revendication 10 ou 11, dans lequel le support est formé par les parois intérieures d'une chambre d'extraction, ladite chambre étant réalisée par photolithographie et gravure, avantageusement gravure DRIE.

13. Procédé d'extraction de composés à cycles aromatiques ayant avantageusement des coefficients de partage octanol/eau supérieurs à 10³ contenus dans une phase liquide, mettant en œuvre un dispositif d'extraction selon l'une des revendications 1 à 7, comportant les étapes:
- mise en contact d'un échantillon liquide comportant au moins un composé aromatique ayant avantageusement un coefficient de partage octanol/eau supérieur à 10³ avec la couche d'absorption.

## Patentansprüche

1. Vorrichtung zur Extraktion von Analyten mit aromatischen Ringen, wobei die Analyte in einer flüssigen Phase enthalten sind, wobei die Extraktionsvorrichtung einen Träger und eine Adsorptionsschicht (7) umfasst, wobei die Adsorptionsschicht poröses Siliziumoxykarbid, SiOxCyHz ist, wobei die Adsorptionsschicht den Träger wenigstens teilweise bedeckt, wobei die Extraktionsvorrichtung derart konfiguriert ist, dass die flüssige Phase in Kontakt mit der Adsorptionsschicht gelangt, wobei der Träger wenigstens durch die Wände einer Extraktionskammer gebildet ist, wobei die Extraktionskammer wenigstens eine Öffnung (6) zur Versorgung mit der flüssigen Phase und wenigstens eine Öffnung (8) zur Ableitung der flüssigen Phase umfasst, wobei die Versorgungs- und die Ableitungsöffnung derart angeordnet sind, dass die flüssige Phase in Kontakt mit der Adsorptionsschicht gelangt, wobei sie von der Versorgungsöffnung in Richtung der Ableitungsöffnung strömt, wobei die Extraktionskammer Mikrostrukturen (9) umfasst, die wenigstens teilweise durch die Adsorptionsschicht aus porösem Siliziumoxykarbid bedeckt sind.

2. Extraktionsvorrichtung nach Anspruch 1, bei der die Analyten mit aromatischen Ringen einen Octanol-Wasser-Verteilungskoeffizient größer als 10³ und vorzugsweise zwischen 10³ und 10⁵ enthalten haben.

3. Extraktionsvorrichtung nach Anspruch 1 oder 2, bei der x zwischen 1 und 2 enthalten ist, vorzugsweise zwischen 1,4 und 1,8; y zwischen 0,8 und 3 enthalten ist, vorzugsweise zwischen 1 uns 2,5; und z zwischen 2,5 und 4,5 enthalten ist, vorzugsweise zwischen 3 und 4,1.

4. Extraktionsvorrichtung nach Anspruch 1, 2 oder 3, bei der die Adsorptionsschicht (7) eine Dicke hat, die zwischen 50 nm und 2000 nm enthalten ist, vorzugsweise zwischen 50 nm und 1000 nm.

5. Extraktionsvorrichtung nach einem der Ansprüche 1 bis 4, bei der die Adsorptionsschicht (7) eine Porosität hat, die zwischen 3 % und 60 % enthalten ist, vorzugsweise zwischen 10 % und 40 %, und Poren umfasst, die einen Radius aufweisen, der zwischen 1 nm und 5 nm enthalten ist.

6. Extraktionsvorrichtung nach einem der Ansprüche 1 bis 5, bei der die Mikrostrukturen (9) Mikrosäulen sind, die die Absorptionsschicht tragen.

7. Extraktionsvorrichtung nach einem der Ansprüche 1 bis 6, umfassend ein Gehäuse, das die Kammer umfasst, und einen Deckel, der die Kammer schließt.

8. Analysesystem, umfassend eine Extraktionsvorrichtung nach einem der Ansprüche 1 bis 7 und eine Vorrichtung zur Analyse der Verbindungen, die durch die Extraktionsvorrichtung extrahiert sind.

9. Analysesystem nach Anspruch 8, bei dem die Analysevorrichtung eine Chromatographiesäule ist, die einem Detektor zugeordnet ist, vorzugsweise eine Chromatographiemikrosäule, die einem Detektor zugeordnet ist.

10. Verfahren zur Herstellung einer Extraktionsvorrichtung nach einem der Ansprüche 1 bis 7, umfassend die Schritte:
a) Realisieren eines Trägers,
b) Bilden einer Schicht aus porösem Siliziumoykarbid, SiOxCyHz, auf dem Träger.

11. Herstellungsverfahren nach Anspruch 10, bei dem der Schritt b) durch chemische Dampfphasenabscheidung realisiert wird, vorzugsweise durch plasmaunterstützte chemische Dampfphasenabscheidung, und/oder wobei während des Schritts b) ein porogenes Agens während der Bildung der Schicht aus porösem Siliziumoxykarbid verwendet wird, beispielsweise Norbornadien, und ein Temperungsschritt zum Entfernen des porogenen Agens stattfindet.

12. Herstellungsverfahren nach Anspruch 10 oder 11, bei dem der Träger durch die Innenwände einer Extraktionskammer gebildet ist, wobei die Kammer durch Photolithographie und Ätzen realisiert ist, vorzugsweise DRIE-Ätzen.

13. Verfahren zur Extraktion von Verbindungen mit aromatischen Ringen, die vorzugsweise Octanol/Wasser-Verteilungskoeffizienten größer als 10³ haben, die in einer flüssigen Phase enthalten sind, unter Verwendung einer Extraktionsvorrichtung nach einem der Ansprüche 1 bis 7, umfassend die Schritte:
- Inkontaktbringen einer flüssigen Probe, die wenigstens eine aromatische Verbindung umfasst, die vorzugsweise einen Octanol/Wasser-Verteilungskoeffizienten größer als 10³ hat, mit der Absorptionsschicht.

## Claims

1. Extraction device for the extraction of analytes with aromatic cycles, said analytes being contained in a liquid phase, said extraction device comprising a support and an adsorption layer (7), said adsorption layer being porous silicon oxycarbide SiOxCyHz, said adsorption layer at least partially covering said support, the extraction device being configured to put into contact the liquid phase and the adsorption layer, in which the support is composed of at least the walls of an extraction chamber, said extraction chamber comprising at least one liquid phase supply orifice (6) and at least one liquid phase outlet orifice (8), said supply and outlet orifices being arranged such that the liquid phase comes into contact with the adsorption layer (7) and flows from the supply orifice (6) towards the extraction orifice (8), said extraction chamber comprising microstructures (9) at least partly coated by the adsorption layer (7) composed of porous silicon oxycarbide.

2. Extraction device according to claim 1, in which the octanol-water partition coefficient of analytes of interest with aromatic cycles is more than 10³, and advantageously between 10³ and 10⁵.

3. Extraction device according to claim 1 or 2, in which x is between 1 and 2 and preferably between 1.4 and 1.8; y is between 0.8 and 3 and preferably between 1 and 2.5 and z is between 2.5 and 4.5 and preferably between 3 and 4.1.

4. Extraction device according to claim 1, 2 or 3, in which the thickness of the adsorption layer (7) is between 50 nm and 2000 nm, and preferably between 50 nm and 1000 nm.

5. Extraction device according to one of claims 1 to 4, in which the porosity of the adsorption layer (7) is between 3% and 60%, and preferably between 10% and 40% and the radius of pores is between 1 nm and 5 nm.

6. Extraction device according to one of claims 1 to 5, in which the microstructures (9) are micropillars supporting the absorption layer.

7. Extraction device according to any one of claims 1 to 6, including a box containing the chamber, and a cover closing the chamber.

8. Analysis system comprising an extraction device according to one of claims 1 to 7 and a device for the analysis of compounds extracted by the extraction device.

9. Analysis system according to claim 8, in which the analysis device is a chromatography column associated with a detector, advantageously a chromatography microcolumn associated with a detector.

10. Method of manufacturing an extraction device according to one of claims 1 to 7, including the following steps:
a) make a support,
b) form a layer of porous silicon oxycarbide SiOxCyHz on the support,

11. Manufacturing method according to claim 10, in which step b) is done by chemical vapour deposition, advantageously plasma-enhanced chemical vapour deposition and/or a porogen, for example norbornadiene, is used in step b) during formation of the porous silicon oxycarbide layer, with an annealing step to eliminate the porogen.

12. Manufacturing method according to claim 10 or 11, in which the support is formed by the internal walls of an extraction chamber, said chamber being made by photolithography and etching, advantageously DRIE etching.

13. Method for extraction of compounds with aromatic cycles that advantageously have octanol/water partition coefficients of more than 10³ contained in a liquid phase, making use of an extraction device according to one of claims 1 to 7, including the following steps:
- bring a liquid sample comprising at least one aromatic compound advantageously having an octanol/water partition coefficient of more than 10³ into contact with the absorption layer.
